# EUROPEAN PATENT APPLICATION

(11) **EP 2 394 982 A1**
(43) Date of publication of application: **14.12.2011**
(21) Application number: 10005903.9
(22) Date of filing: 08.06.2010
(51) Int. Cl.: C07C 209/62, C07D 487/08, C07C 211/36

(54) **Process for the preparation of cis-diaminocycloalkanes**

(71) Applicant: Lonza Ltd, 4052 Basel (CH)
(72) Inventor: Zaragoza Dörwald, Florencio, 3930 Visp (CH); Hanselmann, Paul, 3902 Brig-Glis (CH); Zollinger, Daniel, 3960 Sierre (CH)

(57) **Abstract**

The present invention relates to a process for the preparation of cyclic *cis*-diamines of formula 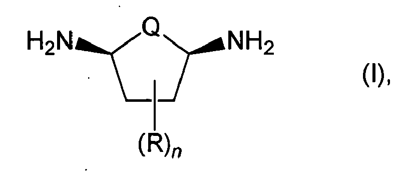 wherein Q is selected from the group consisting of -CH₂-, -CH(CH₃)-, -C(CH₃)₂-, -(CH₂)₂-,-(CH₂)₃- and -(CH₂)₄-;
*n* is an integer from 0 to 4;
and each substituent R, if present, is independently a linear or branched C₁₋₈ alkyl attached to any one of the depicted carbon atoms;
starting from cyclic dienes such as cyclopentadiene and an azodicarboxylic acid derivative such as azodicarboxamide. Also claimed are novel compounds as intermediates in the process.

## Description

The present invention relates to a process for the preparation of cycloaliphatic diamines of general formula 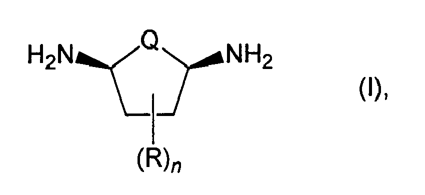 wherein Q, R and *n* are as defined below, and new intermediates in said process.

In particular, the present disclosure describes a process for the preparation of diamines of general formula (I), in which Q represents -CH₂-, -CH(CH₃)-, -C(CH₃)₂-, -(CH₂)₂-, -(CH₂)₃-, or -(CH₂)₄-, *n* is an integer from 0 to 4, and each substituent R, if present, independently represents linear or branched C₁₋₈ alkyl attached to any one of the carbon atoms depicted in formula I. Also disclosed are novel intermediates in the process.

Aliphatic diamines are important intermediates or starting materials in organic syntheses, e. g. as monomers for the preparation of polyamides, as building blocks for the preparation of ligands for metal complexes, or for the preparation of biologically active compounds. For instance, 1,4-diaminobutane (putrescine) has been successfully used for the preparation of ligands for alkali metal ions (J. Am. Chem. Soc. 1973, 95, 3569-3572) and is also being used as monomer for the preparation of polyamides (e.g. Stanyl^{®}, polyamide of diaminobutane and adipic acid). Furthermore, numerous drugs contain a diamine substructure and require such a diamine for their manufacture, e.g. ciprofloxacin, chloroquine, or trovafloxacin.
*cis*- 1, 3-Diaminocyclopentane has been used as intermediate for the preparation of new agents for the inhibition of cell proliferation (WO 2007/138277), new antimalarials (US 5 736 557), and new quinolonecarboxylic acid derivatives as antibacterials (US 5 519 024).

If diamines such as 1,4-diaminobutane are intended to be used for the preparation of polymers such as polyamides, the purity of the diamine will be of critical importance. Small amounts of monoamines will lead to an interruption of the polycondensation process, and will thus limit the length of the polymer chains and have a deleterious effect on the mechanical stability of the final polymer.

Currently, the preferred method for the preparation of 1,4-diaminobutane, 1,6-diaminohexane, and related α,ω-diamines is the hydrogenation of the corresponding dinitriles. This hydrogenation is, however, difficult, because imines are formed as intermediates, which may hydrolyze or cyclize, and thereby lead to the formation of monoamines or other byproducts. Furthermore, the imines can also react with already present amino groups, leading to the formation of secondary amines. Thus, the hydrogenation of succinodinitrile (NC-CH₂-CH₂-CN) usually gives pyrrolidine as well as 1,4-diaminobutane. Therefore, new methods for the preparation of diamines which are not based on the hydrogenation of dinitriles and, because of the reaction mechanism, cannot lead to the formation of monoamine byproducts will be highly valuable.

Cyclic diamines of general formula (I) are conformationally constrained analogs of 1,4-diaminobutane, and their derivatives may show properties similar to those of the corresponding derivatives of 1,4-diaminobutane.

Various preparations of *cis*-1,3-diaminocyclopentane have been reported in the literature. In WO 2007/138277 this compound is prepared by hydrogenation of 2,3-diazabi-cyclo[2.2.1]heptane dihydrochloride in aqueous ethanol in the presence of platinum on charcoal. This yields the dihydrochloride of *cis*-1,3-diaminocyclopentane, which must be deprotonated by treatment with a base, and separated from the resulting salts by filtration or extraction. The required 2,3-diazabicyclo[2.2.1]heptane was prepared by Diels-Alder reaction of cyclopentadiene with diethyl azodicarboxylate, followed by hydrogenation, base-catalyzed hydrolysis of the ethyl carbamate groups, protection by treatment with di-*tert* butyl dicarbonate (Boc₂O), and acid-mediated cleavage of the Boc-groups ( Tetrahedron Lett. 2002, 43, 5551-5554). Alternatively, *cis*-1,3-diaminocyclopentane may be prepared from 2,3-diazabicyclo[2.2.1]heptane-2,3-dicarboxylate by treatment with sodium in liquid ammonia (US 5 736 557). The required starting material can be prepared from the hydrogenated Diels-Alder adduct of diethyl azodicarboxylate and cyclopentadiene by treatment with potassium hydroxide in ethylene glycol (*Org. Synth. Coll. Vol. V* **96**).

The methods described in the literature for the preparation of *cis*-1,3-diaminocyclopentane are wasteful, because they require base-catalyzed hydrolysis of a carbamic acid ester to yield a carbamate salt, which must be acidified in order to undergo decarboxylation. To obtain the free, non-protonated 2,3-diazabicyclo[2.2.1]heptane, an additional treatment with base is required. Moreover, diethyl azodicarboxylate and related azodicarboxylic esters are toxic, shock sensitive, thermally unstable, and relatively expensive.

Thus, a cheap, simple process for the preparation of *cis*-1,3-diaminocyclopentane and related diamines has not yet been reported, and would be highly valuable. If such a process would be based on the hydrogenolytic N-N-bond cleavage of a 2,3-diazabi-cyclo[2.2.1]heptane derivative or a 2,3-diazabicyclo[2.2.1]heptene, then no monoamine byproducts should be formed, and highly pure *cis*-diamines, well suited for the preparation of polyamides, should be obtained as products.

### DEFINITIONS

"Alkyl" refers to a monovalent radical formally derived from a saturated, linear, branched, or cyclic hydrocarbon, such as methyl, ethyl, 1-propyl, 2-propyl, butyl, hexyl, octyl, decyl, cyclopropyl, cyclobutyl, cyclopentyl, cyclohexyl, and the like.

"Lower alkyl", in particular C₁₋₈ alkyl, refers to a monovalent radical formally derived from a saturated, linear, branched, or cyclic C₁₋₈ hydrocarbon, such as methyl, ethyl, propyl, 2-propyl, cyclopropyl, butyl, isobutyl, cyclobutyl, and the like. Accordingly, C₁₋₆ alkyl refers to a monovalent radical formally derived from a saturated, linear, branched, or cyclic C₁₋₈ hydrocarbon.

The term "*cis*'' refers to the relative stereochemical configuration of two substituents in the same molecule. More specifically, "*cis*" means that the two substituents the term refers to are located on the same side of a ring (i. e., a plane defined by the ring atoms) or a double bond.

The expression "one-pot synthesis" means a two- or multistep synthetic process, wherein at least one of the intermediates occurring in the course of the process is neither purified nor isolated, but directly used as starting material for a subsequent step. However, the expression "one-pot synthesis" does not necessarily imply that two or all steps are actually conducted in the same reaction vessel.

According to the invention, *cis*-diamines of formula 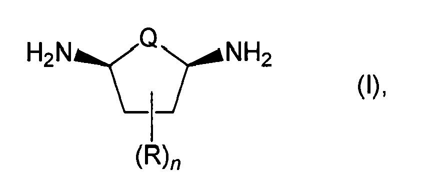 wherein
Q is selected from the group consisting of -CH₂-, -CH(CH₃)-, -C(CH₃)₂-, -(CH₂)₂-, -(CH₂)₃- and -(CH₂)₄-,
*n* is an integer from 0 to 4,
and each substituent R, if present, independently is a linear or branched C₁₋₈ alkyl attached to any one of the depicted carbon atoms,
are prepared by a process comprising the steps of
(i) reacting a cyclic diene of formula 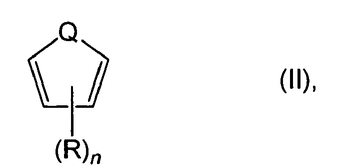 wherein Q, R and *n* are as defined above,
   with an azodicarboxylic acid derivative of formula 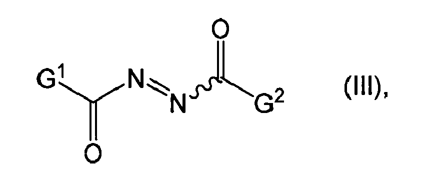 wherein G¹ and G² independently are -NR¹R² or -OR³, R¹ and R² independently being hydrogen or C₁₋₆ alkyl and R³ being C₁₋₈ alkyl,
   to yield a product of formula 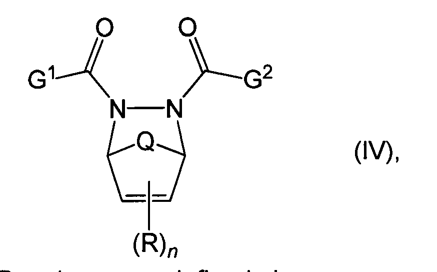 wherein G¹, G², Q, R and *n* are as defined above,
(ii) catalytically hydrogenating the compound of formula IV to obtain a compound of formula 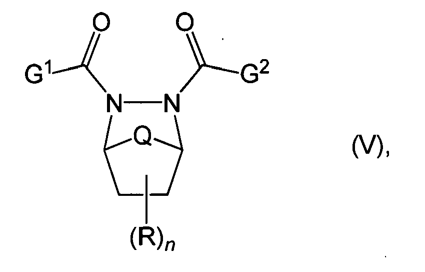 wherein G¹ , G², Q, R and *n* are as defined above,
(iii) reacting the compound of formula V with elemental zinc in the presence of an acid to obtain a compound of formula 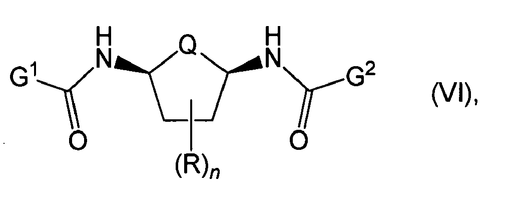 wherein G¹ , G², Q, R and *n* are as defined above,
(iv) hydrolyzing the compound of formula VI with a strong acid to obtain the *cis*-diamine of formula I, and, optionally,
(v) liberating the *cis*-diamine of formula I by treating its salt with a strong base.

Applicants have surprisingly found that azodicarboxamide readily undergoes a Diels-Alder-type cycloaddition with cyclopentadiene, preferably in polar protic solvents such as water or acetic acid, to form 2,3-bis(aminocarbonyl)-2,3-diazabicyclo[2.2.1]hept-2-ene in high yield.

Azodicarboxamide (H₂N-CO-N=N-CO-NH₂), is a cheap compound that is used e. g. as food additive, flour-bleaching agent, and foaming agent for the production of foamed plastics. Because of its lower price, lower toxicity, and higher stability it may be a more suitable starting material for the 1,4-diamination of 1,3-dienes than azodicarboxylic esters. Unfortunately, azodicarboxamide is only slightly soluble in DMF or DMSO, and practically insoluble in most other organic solvents. Only few examples of organic reactions with this compound have been reported, and most of them were conducted in DMF.

Step (i) of the process of the invention can be performed at room temperature or at higher temperatures, such as 40 °C or higher, depending on the reactivity and stability of the starting materials and on the solvent. If a volatile 1,3-diene is used and the reaction is to be performed above the boiling point of said diene, an autoclave or another closed vessel must be used as reactor. Suitable dienes which may be used as starting materials in the process of the invention include cyclopentadiene, methylcyclopentadiene, other alkylated cyclopentadienes, 1,3-cyclohexadiene, alkylated cyclohexadienes, 1,3-cycloheptadiene, or 1,3-cyclooctadiene. Some non-conjugated dienes, such as 1,4-cyclohexadiene, 1,4-cycloheptadiene, or 1,5-cyclooctadiene, may rearrange *in situ* into the corresponding conjugated 1,3-dienes under the conditions of the Diels-Alder reaction, and then undergo the normal cycloaddition. Therefore, such unconjugated cyclic dienes are also included within the scope of the present invention.

As will be evident to those skilled in the art upon reading the present disclosure, the zinc-mediated hydrogenolysis of intermediate V will also be applicable to the hydrogenated Diels-Alder products of dienes and azodicarboxylic esters, to yield the corresponding *cis*-bis(alkoxycarbonylamino)cycloalkanes (VI, G¹ = G² = -OR³), depending on the azodicarboxylic acid ester used as starting material. Accordingly, the present invention also provides a process for the preparation of intermediates (IV, V and VI) and diamines I from dienes and azodicarboxylic esters.

The cycloaddition products of general formula (IV) can be readily hydrogenated in the presence of hydrogenation catalysts suitable for the hydrogenation of carbon-carbon double bonds, such as nickel-, platinum-, or palladium-based catalysts, in particular Raney nickel or palladium on charcoal, and using standard reaction conditions, e. g. hydrogen pressures of 1-50 bar and reaction temperatures in the range of 20-70 °C. Surprisingly, however, also this reaction can be conducted in water or acetic acid, and isolation and drying of the cycloaddition product (IV) is therefore not required. Thus, when water is used as solvent in step (i), a simple washing of the reaction mixture of the Diels-Alder reaction with a water-immiscible organic solvent, such as toluene, ethyl acetate or dichloromethane, in which the cycloaddition product (IV) is not or only sparingly soluble, will remove unreacted cyclopentadiene and any dicyclopentadiene present as impurity or byproduct, and yield an aqueous suspension of (IV) that can be directly hydrogenated.

It has further been found that the N-N single bond of the hydrogenated intermediate (V) can be reductively cleaved by treatment with zinc in the presence of an acid to yield a *cis*-diureido or *cis*-bis(alkoxycarbonylamino) intermediate (VI). Suitable acids are, for example, carboxylic acids such as formic, acetic, trifluoroacetic, trichloroacetic, propionic, butyric, glutaric, or valeric acid, or mineral acids, such as hydrochloric, phosphoric, or sulfuric acid. The ureido or alkoxycarbonylamino moieties of the intermediate VI can be cleaved by treatment with a strong acid, such as hydrochloric, hydrobromic, sulfuric, trichloroacetic, tetrafluoroboric (HBF₄), or perchloric acid, to yield a salt of the diamine (I) and the respective strong acid. Said salt can be isolated as such or treated with a suitable base, such as an alkali or alkaline earth hydroxide, or silver hydroxide if a hydrohalic acid has been used as strong acid, to give the free base (I).

In a preferred embodiment, steps (iii) and (iv) of the process of the invention are conducted without isolating the intermediate compound of formula VI, i. e. by directly adding a strong acid to the reaction mixture resulting from step (iii).

It is, of course, also possible and within the scope of the invention to isolate the intermediate compound VI according to standard work-up methods, such as extraction, followed by evaporation of the solvent and recrystallization, or direct filtration and recrystallization.

The salts of the diamines of general formula (I) and strong acids can be isolated and, if required, purified by conventional methods, for example by filtering off and recrystallizing from a polar solvent.

Diamines such as I may be separated from water, dried and purified by saturating an aqueous solution of said diamine or a salt thereof with a strong base such as sodium hydroxide, whereupon the diamine usually separates as a second liquid phase. The diamine may be separated by decantation or by extraction with a suitable solvent such as diethyl ether, and dried and purified further by treatment with solid sodium hydroxide or potassium hydroxide, followed by distillation (Woodburn, O'Gee, J. Org. Chem. 1952, 17, 1235-1244; Denk et al., Tetrahedron 2003, 59, 7565-7570).

As mentioned above, step (i) is preferably conducted in a polar protic solvent.

More preferably, the polar protic solvent is selected from the group consisting of water, C₁₋₄ alkanols, C₂₋₄ alkanoic acids, and mixtures of water and one or more C₁₋₄ alkanols or C₂₋₄ alkanoic acids. The expression "C₁₋₄ alkanols" includes compounds having one or more hydroxy groups and 1 to 4 carbon atoms, such as methanol, ethanol, 1-propanol, 2-propanol (isopropyl alcohol), 1-butanol, 2-butanol (*sec*-butyl alcohol), 2-methyl-1-propanol (isobutyl alcohol), 2-methyl-2-propanol (*tert-*butyl alcohol), 1,2-ethanediol (ethylene glycol), 1,2-propanediol, 1,3-propanediol, 1,2-butanediol, 1,3-butanediol, 1,4-butanediol, 2,3-butanediol, and 1,2,3-propanetriol (glycerol). The expression "C₂₋₄ alkanoic acids" includes compounds such as acetic (ethanoic) acid, propionic (propanoic) acid, butyric (butanoic) acid and isobutyric (2-methylpropanoic) acid.

Still more preferably, the polar protic solvent in step (i) is water.

Most preferably, the solvent in steps (i), (ii) and (iv) is water.

As mentioned above, the hydrogenation catalyst in step (ii) may be any catalyst known in the art to catalyze the hydrogenation of carbon-carbon double bonds.

In a preferred embodiment the hydrogenation catalyst in step (ii) is Raney nickel.

The acid in the zinc-mediated hydrogenolysis step (iii) is preferably acetic acid.

The strong acid in the cleavage step (iv) is preferably hydrochloric acid.

The process of the invention is particularly suited to the preparation of *cis*-diamines of formula I, wherein Q is a methylene (-CH₂-) group. The cyclic diene (II) starting material in this embodiment is a cyclopentadiene.

In another preferred embodiment *n* is zero, the cyclic diene (II) being an unsubstituted cycloalka-1,3-diene.

In still another preferred embodiment, G¹ and G² in the azodicarboxylic acid derivative (III) are -NR¹R² moieties wherein R¹ and R² are hydrogen, G¹ and G² thus being amino (-NH₂) groups and the azodicarboxylic acid derivative being azodicarboxamide.

As mentioned above, it is also possible to produce the cyclic diene in situ by rearrangement of a corresponding non-conjugated cyclic diene, for example from 1,4-cyclohexadiene that can rearrange to 1,3-cyclohexadiene before undergoing the cycloaddition step (i).

The intermediate compounds of formula 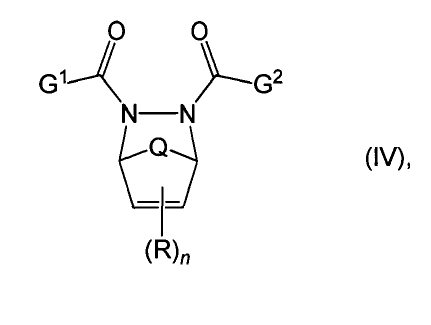 wherein G¹ and G² are independently -NR¹R² with R¹ and R² independently being hydrogen or C₁₋₆ alkyl; Q is selected from the group consisting of -CH₂-, -CH(CH₃)-, -C(CH₃)₂-, -(CH₂)₂-, -(CH₂)₃- and -(CH₂)₄-, *n* is an integer from 0 to 4, and each substituent R, if present, independently is a linear or branched C₁₋₈ alkyl attached to any one of the depicted carbon atoms, are novel and also an object of the present invention.

The intermediate compounds of formula 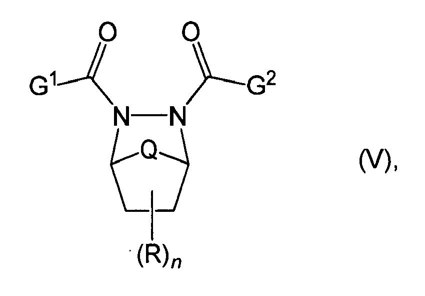 wherein G¹ and G² are independently -NR¹R² with R¹ and R² independently being hydrogen or C₁₋₈ alkyl; Q is selected from the group consisting of -CH₂-, -CH(CH₃)-, -C(CH₃)₂-, -(CH₂)₂-, -(CH₂)₃- and -(CH₂)₄-, *n* is an integer from 0 to 4, and each substituent R, if present, independently is a linear or branched C₁₋₈ alkyl attached to any one of the depicted carbon atoms, are likewise novel and also an object of the present invention.

The intermediate compounds of formula 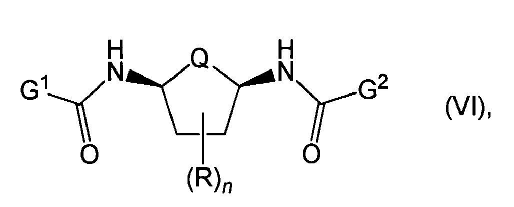 wherein G¹ and G² are independently -NR¹R² with R¹ and R² independently being hydrogen or C₁₋₈ alkyl; Q is selected from the group consisting of -CH₂-, -CH(CH₃)-, -C(CH₃)₂-, -(CH₂)₂-, -(CH₂)₃- arid -(CH₂)₄-, *n* is an integer from 0 to 4, and each substituent R, if present, independently is a linear or branched C₁₋₈ alkyl attached to any one of the depicted carbon atoms are also novel and an object of the present invention.

In particularly preferred embodiments of the intermediates IV, V and VI, both R¹ and R² are hydrogen, Q is -CH₂-, and *n* is zero.

### EXAMPLES

The following examples are intended to illustrate the present invention, but not to limit its scope in any way. The products were identified by ¹H NMR spectroscopy.

### Example 1

### 2,3-Bis(aminocarbonyl)-2,3-diazabicyclo[2.2.1]hept-5-ene

To a stirred suspension of azodicarboxamide (245.8 g, 2.12 mol) in water (870 ml) at 38 °C was dropwise added cyclopentadiene (100 g, 1.51 mol) within 0.5 h. The resulting mixture was stirred at 38 °C for 24 h, and then more cyclopentadiene (100 g, 1.51 mol) was added within 0.5 h. The mixture was stirred at 38 °C for another 24 h, to yield an almost colorless suspension of 2,3-bis(aminocarbonyl)-2,3-diazabicyclo[2.2.1]-hept-5-ene. Filtration and washing with toluene gave the wet title compound (488 g), which was used in the next step without further purification.

A sample was dried under reduced pressure to yield the title compound as a slightly yellow solid, mp 195-198 °C (decomposition).

¹H NMR (400 MHz, d₆-DMSO): δ 1.45 (d, J= 8 Hz, 1 H), 1.58 (d, J= 8 Hz, 1 H), 5.01 (br. s, 2H), 6.47 (br. s, 6H).

### Example 2

### 2,3-Bis(aminocarbonyl)-2,3-diazabicyclo[2.2.1]hept-5-ene

To a stirred suspension of azodicarboxamide (13.9 g, 0.12 mol) in water (76.3 ml) at 70 °C was added cyclopentadiene (9.5 g, 0.14 mol) at a constant rate (0.43 g/min) within 22 min and at a maximum pressure of 2.7 bar. The reaction mixture was stirred at 70 °C for 95 min. The resulting suspension was cooled to room temperature and the solid filtered off and washed with water (109.4 ml). After filtration, a wet cake was obtained (17.45 g). By ¹H NMR it was determined that the wet cake contained 81% of 2,3-bis(aminocarbonyl)-2,3-diazabicyclo[2.2.1]hept-5-ene (14.1 g, 77 mmol), corresponding to a yield of 65%. The rest of the cake was unreacted starting material (11 %) and water (8%).

When acetic acid was used instead of water, a comparable result could be obtained.

### Example 3

### 2,3-Bis(aminocarbonyl)-2,3-diazabicyclo[2.2.1]hept-5-ene

To a stirred suspension of azodicarboxamide (13.9 g, 0.12 mol) and Tween® 80 (Polysorbate 80, 1.59 g, 1.2 mmol) in water (215.8 ml) at 70 °C was added cyclopentadiene (9.5 g, 0.14 mol) at a constant rate (0.56 g/min) within 17 min and at a maximum pressure of 2.1 bar. The reaction mixture was stirred at 70 °C for 35 min. The resulting suspension was cooled to room temperature within 35 min and the solid was filtered off and washed with water (71 ml). After filtration, a wet cake was obtained (16.6 g). By ¹H NMR it was determined that the wet cake contained 95% of 2,3-bis(aminocarbonyl)-2,3-diazabicyclo[2.2.1]hept-5-ene (15.8 g, 87 mmol), corresponding to a yield of 72%. The rest of the cake was water (5%).

### Example 4

### 2,3-Bis(aminocarbonyl)-2,3-diazabicyclo[2.2.1]hept-5-ene

Azodicarboxamide (14.3 g, 0.12 mol) was dissolved in glycerol (161.32 g) and at 70 °C cyclopentadiene (10 g, 0.15 mol) was added at a constant rate (0.67 g/min) within 15 min and at a maximum pressure of 2.4 bar. The resulting mixture was stirred at 70 °C for 60 min. Then the reactor was cooled to room temperature within 60 min. The resulting viscous solution could be filtered upon addition of water (400 g). After filtration, a wet cake was obtained (21.4 g). By 1H NMR it was determined that the wet cake contained 72% of 2,3-bis(aminocarbonyl)-2,3-diazabicyclo[2.2.1]hept-5-ene (15.4 g, 85 mmol), corresponding to a yield of 68%.

### Example 5

### 2,3-Bis(aminocarbonyl)-2,3-diazabicyclo[2.2.1]heptane

To a suspension of 2,3-bis(aminocarbonyl)-2,3-diazabicyclo[2.2.1]hept-5-ene (10 g, 54.9 mmol) in butanol (80 ml) was added Raney nickel (3.0 g), and the resulting mixture was hydrogenated at 50 °C for 15 min at 50 bar and for 10 min at 90 bar. The resulting suspension was diluted with acetic acid (140 ml), filtered, and the filtrate was concentrated under reduced pressure to yield 9.1 g (90%) of the title compound as a solid.

¹H NMR (400 MHz, d₆-DMSO): δ 1.42 (br d, *J*= 8 Hz, 1 H), 1.47 (d, *J*= 8 Hz, 1 H), 1.60 (m, 4H), 4.39 (m, 2H), 5.90 (m, 1 H), 6.39 (br. s, 3H).

### Example 6

### cis-1,3-Diaminocyclopentane

A mixture of 2,3-bis(aminocarbonyl)-2,3-diazabicyclo[2.2.1]heptane (50.3 g, 0.27 mol), acetic acid (784 g) and zinc dust (<10 µm, 124 g, 2.07 mol) was stirred at reflux temperature for 5 days to obtain *cis*-1,3-diureidocyclopentane (VI, Q¹ = Q² = NH₂, *n*= 0). The acetic acid was evaporated under reduced pressure, and to the residue was added concentrated aqueous hydrochloric acid (907 g). The mixture was heated to reflux and 553 g of liquid was distilled off while more concentrated hydrochloric acid (502 g) was added. Refluxing was continued until NMR-analysis indicated complete conversion to *cis*-1,3-diaminocyclopentane dihydrochloride. The mixture was concentrated to dryness, and to the residue were added THF (654 g) and solid sodium hydroxide (414 g) in portions. When the exothermicity had subsided the mixture was filtered, the solid washed extensively with THF, and the combined THF extracts were concentrated under reduced pressure to yield 14.6 g (54%) of the crude title amine.

¹H NMR (400 MHz, d₆-DMSO): δ 1.14 (m, 1H), 1.43 (m, 2H), 1.71 (m, 2H), 1.95 (m, 1H), 3.21 (m, 2H).

## Claims

1. A process for the preparation of a *cis*-diamine of formula 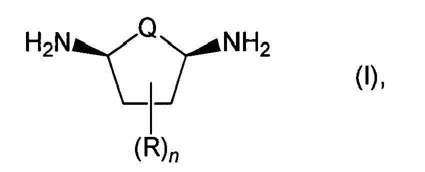 or a salt thereof,
wherein
Q is selected from the group consisting of -CH₂-, -CH(CH₃)-, -C(CH₃)₂-, -(CH₂)₂-, -(CH₂)₃- and -(CH₂)₄-,
*n* is an integer from 0 to 4,
and each substituent R, if present, independently is a linear or branched C₁₋₈ alkyl attached to any one of the depicted carbon atoms,
said process comprising the steps of
(i) reacting a cyclic diene of formula 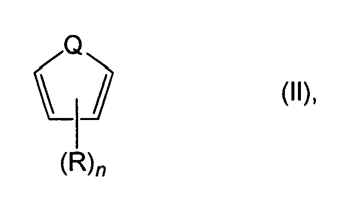 wherein Q, R and *n* are as defined above,
with an azodicarboxylic acid derivative of formula 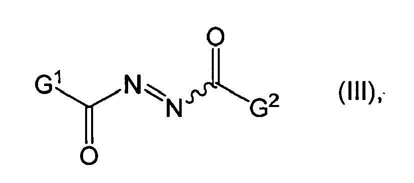 wherein G¹ and G² independently are -NR¹R² or -OR³, with R¹ and R² independently being hydrogen or C₁₋₈ alkyl, and R³ being C₁₋₈ alkyl,
to yield a product of general formula 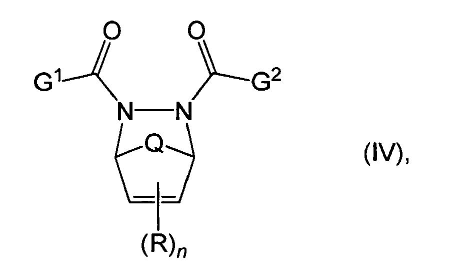 wherein G¹ , G², Q, R and *n* are as defined above,
(ii) catalytically hydrogenating the compound of formula IV to obtain a compound of formula 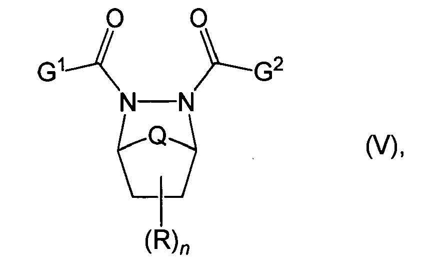 wherein G¹ G², Q, R and *n* are as defined above,
(iii) reacting the compound of formula V with zinc in the presence of a carboxylic acid to obtain a compound of formula 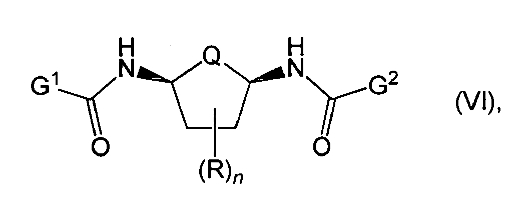 wherein Q, G¹, G², R and *n* are as defined above,
(iv) hydrolyzing the compound of formula VI with a strong acid to obtain the salt of the strong acid and the *cis*-diamine of formula I, and, optionally,
(v) liberating the *cis*-diamine of formula I by treating its salt with a strong base.

2. The process of claim 1, wherein steps (iii) and (iv) are conducted as a one-pot process.

3. The process of claim 1 or 2, wherein step (i) is conducted in a polar protic solvent.

4. The process of claim 3, wherein the polar protic solvent is selected from the group consisting of water, C₁₋₄ alkanols, C₂₋₄ alkanoic acids, and mixtures of water and one or more of the beforementioned.

5. The process of claim 4, wherein the polar protic solvent is water.

6. The process of any of claims 1 to 5, wherein the hydrogenation in step (ii) is carried out using Raney nickel as hydrogenation catalyst.

7. The process of any of claims 1 to 6, wherein the carboxylic acid in step (iii) is acetic acid.

8. The process of any of claims 1 to 7, wherein the strong acid in step (iv) is hydrochloric acid.

9. The process of any of claims 1 to 8, wherein Q is -CH₂-.

10. The process of any of claims 1 to 9, wherein *n* is zero.

11. The process of any of claims 1 to 10, wherein G¹ and G² are -NR¹R² with R¹ and R² being hydrogen.

12. A compound having the formula 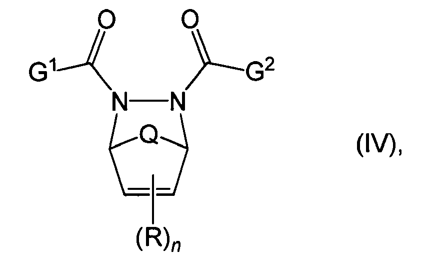 wherein G¹ and G² are independently -NR¹R² with R¹ and R² independently being hydrogen or C₁₋₆ alkyl; and Q, R and *n* are as defined in claim 1.

13. A compound having the formula 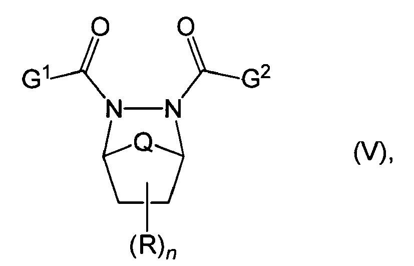 wherein G¹ and G² are independently -NR¹R² with R¹ and R² independently being hydrogen or C₁₋₆ alkyl; and Q, R and *n* are as defined in claim 1.

14. A compound having the formula 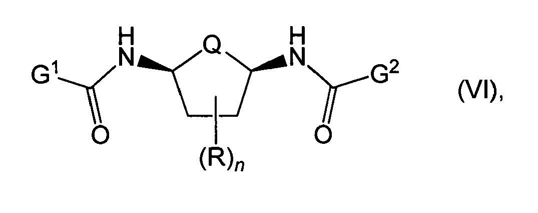 wherein G¹ and G² are independently -NR¹R² with R¹ and R² independently being hydrogen or C₁₋₈ alkyl; and Q, R and *n* are as defined in claim 1.

15. The compound of claim 12, 13 or 14, wherein R¹ and R² are hydrogen, Q is -CH₂-, and *n* is zero.
